Europäisches Patentamt

European Patent Office

Office européen des brevets

(19)

(11) **EP 0 612 259 B1**

(12) **EUROPÄISCHE PATENTSCHRIFT**

(45) Veröffentlichungstag und Bekanntmachung des
Hinweises auf die Patenterteilung:
**20.11.1996 Patentblatt 1996/47**

(21) Anmeldenummer: **92919908.1**

(22) Anmeldetag: **14.09.1992**

(51) Int. Cl.$^6$: **A61N 1/32**

(86) Internationale Anmeldenummer:
**PCT/DE92/00801**

(87) Internationale Veröffentlichungsnummer:
**WO 93/09843 (27.05.1993 Gazette 1993/13)**

(54) **ELEKTROTHERAPIE-GERÄT**

**ELECTROTHERAPY APPARATUS**

**APPAREIL D'ELECTROTHERAPIE**

(84) Benannte Vertragsstaaten:
**AT CH DE FR GB IT LI**

(30) Priorität: **15.11.1991 DE 4137579**

(43) Veröffentlichungstag der Anmeldung:
**31.08.1994 Patentblatt 1994/35**

(73) Patentinhaber: **SCHÖNDORF, Erhard**
**D-66111 Saarbrücken (DE)**

(72) Erfinder: **SCHÖNDORF, Erhard**
**D-66111 Saarbrücken (DE)**

(74) Vertreter: **Schickedanz, Willi, Dipl.-Ing.**
**Langener Strasse 68**
**63073 Offenbach (DE)**

(56) Entgegenhaltungen:
**EP-A- 0 035 138          WO-A-90/11798**
**CH-A- 624 850          DE-A- 3 010 716**
**FR-A- 992 688          FR-A- 2 570 529**
**US-A- 4 535 777**

• **A.H. Seidman, "Integrated Circuits Applications**
**Handbook", John Wiley & Sons, 1983, Seiten 387**
**bis 389**

# Beschreibung

Die Erfindung betrifft ein Elektrotherapie-Gerät nach dem Oberbegriff des Patentanspruchs 1.

Elektrotherapeutische Geräte werden auf vielfältige Weise für die Behandlung von Muskeln und Nerven eingesetzt. Dabei wird von dem Umstand Gebrauch gemacht, daß Gewebe in der Regel nicht elektrisch neutral ist, sondern elektrische Ladungen in Form von Ionen trägt. Um Gewebe auf nicht-mechanische Weise zu beeinflussen, müssen elektrische oder magnetische Felder auf die Ionen einwirken, wodurch Kräfte entstehen. Die Art der hierbei auftretenden Kräfte wird wesentlich durch die Form, die Amplitude und den zeitlichen Verlauf der applizierten Felder bestimmt. Wird ein Gewebe einem elektrischen Gleichfeld ausgesetzt, so fließen auf der einen Seite ständig Ionen von diesem Gewebe ab und auf der anderen Seite wieder zu. Da durch den Abbau von Ionen chemische Bindungen aufgebrochen werden können, verändert das Gewebe bei dauerndem Einwirken von Gleichstrom seine Struktur. Findet dagegen ein schneller Polaritätswechsel des angelegten Feldes statt, sei es aufgrund einer sinusförmigen Wechselspannung, sei es aufgrund von positiven oder negativen Spannungsimpulsen, so treten bei bestimmten Frequenzen keine bleibenden Veränderungen des Gewebes mehr auf, weil die Ionen sich nicht sehr weit von ihrer ursprünglichen Position wegbewegen. Sie pendeln dann gewissermaßen m diese ursgrüngliche Position und bewirken u. a. eine Erwärmung des Gewebes.

Um bestimmte therapeutische Effekte Zu erzielen, ist es bereits bekannt, Muskeln oder Nerven elektrischen und/oder magnetischen Feldern von vorgegebener Frequenz und Amplitude auszusetzen (J. Low und A. Reed: Electrotherapy Explained, Butterworth-Heinemann Ltd., 1990, S. 28 bis 42, ISBN 0-07 506 00497). Dabei wird zwischen Spannungen bzw. Strömen unterschieden, die eine Länge oder Dauer von mehr als 1 ms bzw. eine Dauer von weniger als 1 ms haben. Außerdem unterscheidet man Ströme oder Spannungen, die sich allmählich oder abrupt ändern. Die sich allmählich ändernden elektrischen Größen werden wiederum in sinusförmige, diadynamische, "russische", interferierende und hochfrequente Größen unterteilt.

Für die Behandlung von schlaffen Lähmungen ist bereits ein Reizstromgerät bekannt, das mittels einer Elektrode an die Haut eines Patienten angelegt wird (DE-A-29 08 365). Dieses Reizstromgerät sendet zunächst für eine bestimmte Zeit eine unipolare Folge von Impulsen einer ersten Polarität aus, um dann die Polarität umzukehren und eine unipolare Folge von Impulsen einer zweiten Polarität auszusenden. Der Gleichstrommittelwert der beiden Impulsfolgen ist hierbei Null. Zwischen den Impulsfolgen können Pausen von vorgegebenen Längen eingefügt werden. Mit den erwähnten Impulsfolgen soll erreicht werden, daß die Hautreizung, die sich aufgrund einer Säurebildung zwischen Haut und Elektroden ergeben kann, stark reduziert wird. Ein ähnliches Reizstromgerät ist auch aus der Patentanmeldung PCT/US 90/01694 (WO 90/11798) bekannt.

Es ist weiterhin eine Vorrichtung zur Elektrostimulation bekannt, mit der die Milchdrüsen von Kühen und anderen Säugetieren behandelt werden, um die Milchleistung und den Fettgehalt der Milch zu erhöhen und um den Melkvorgang zu verkürzen (DE-C-29 29 293). Diese Vorrichtung weist einen Frequenzmodulator und eine Polaritäts-Einstelleinrichtung auf, womit sich Impulse bestimmter Form erzeugen lassen. Insbesondere können Rechteckimpulse von vorgegebener Länge und Polarität erzeugt werden. Eine ähnliche Vorrichtung, mit der unterschiedliche Kurven- und Impulsformen erzeugt werden können, ist auch aus der FR-A-2 570 529 bekannt.

Um glatte Muskeln und Gefäßgewebe zu stimulieren und zu steuern, ist auch eine programmierbare elektrische Vorrichtung bekannt, mit der nacheinander positive und negative Impulse oder Impulsgruppen erzeugt werden können (EP-C-0 137 007 = AT-E-47 796). Die Impulse steigen hierbei mit einer Zeitkonstanten von etwa 40 Millisekunden an und fallen mit derselben Zeitkonstanten wieder ab, wobei die Impulsbreite etwa 3 ms beträgt. Die einstellbaren Amplituden der Impulse liegen zwischen + 130 V und - 130 V, während die Perioden vorzugsweise bei 625 ms liegen.

Bei einem anderen bekannten Gerät zur Elektrostimulation von Nerven und Muskeln werden Impulse erzeugt, deren Form und Frequenz an die Eigenschaften der sogenannten langsamen Muskelfasern angepaßt sind (EP-C-0 197 889 = AT-E-52 927). Die Frequenz und die Dauer der Impulsfolgen können hierbei geregelt werden.

Neben Gleich-, Impuls- und einfachen Wechselströmen werden auch modulierte Wechselströme für die Elektrotherapie verwendet. Unter den modulierten Wechselströmen ist insbesondere der sogenannte Interferenzstrom bekannt, der auch Schwebungs- oder Nemec-Strom genannt wird (Otto Steuernagel, Skripten zur Elektrotherapie, 3. Aufl., 1976, Band II, S. 6, Selbstverlag des Autors). Man unterscheidet hierbei endogene und exogene Interferenzströme. Bei den endogenen Interferenzströmen werden zwei Mittelfrequenzen außerhalb des menschlichen Körpers erzeugt, die sich erst im Körper überlagern. Dagegen werden die exogenen Interferenzströme außerhalb des Körpers erzeugt und als schon überlagerte Ströme auf Elektroden gegeben, die an den Patienten gelegt werden.

Der Grundgedanke der Interferenzstromtherapie besteht darin, mit zwei sich einander überlagernden bzw. sich kreuzenden Feldern in der Tiefe des Körpers eine Reizwirkung zu erzielen und zugleich die Stromdichte an der Haut niedrig zu halten. Die Überlagerung von zwei voneinander nicht zu sehr abweichenden Frequenzen führt dazu, daß je nach Phasenlage diese beiden Ströme sich zu einem gewissen Zeitpunkt verstärken und zu einem anderen Zeitunkt wieder aufheben (H. Jantsch und F. Schuhfried: Niederfrequente Ströme zur Diagnostik und Therapie, 1974, S. 147).

Es ist ferner ein Interferenzstrom-Therapiegerät bekannt, bei dem drei Wechselströme im Mittelfrequenzhereich Von ca. 4 kHz überlagert werden, die sich hinsichtlich ihrer Frequenzen nur um jeweils einen geringen Betrag voneinander unterscheiden, so daß sich bei der Überlagerung Schwebungen ergeben (DE-A-30 10 716). Die aufgrund der Überlagerung resultierenden Stromkurven haben die Form von sinusförmigen Schwebungs-Hüllkurven auf beiden Seiten der Potential-Nullinie, wobei sich Sinushalbwellen mit großen und mit kleinen Amplituden abwechseln, vergleichbar einer Zweiseitenband-Amplitudenmodulation mit unterdrücktem Träger (vgl. Meinke-Gundlach: Taschenbuch der Hochfrequenztechnik, 4. Auflage, Band 3, Systeme, 1986, O 3, Bild 3, Darstellung im Zeitbereich) . Im Mittel heben sich die einander gegenüberliegenden Halbwellen der Schwebungsbäuche zu jedem Zeitpunkt potentialmäßig auf, d. h. es ergibt sich keine Gleichstromkomponente. Allerdings liegen die beiden Elektroden, die an einen zu behandelnden Muskel herangeführt werden, stets auf Potential, so daß immer ein zur Nullinie symmetrischer Wechselstrom fließt. Die Muskel wird somit ortsmäßig stets auf die gleiche Weise behandelt; ein Wechsel der bevorzugten Behandlungsrichtung während einer vorgegebenen Zeit findet nicht statt. Hierdurch entsteht oft eine Muskelverkrampfung, die der Patient als unangenehm empfindet.

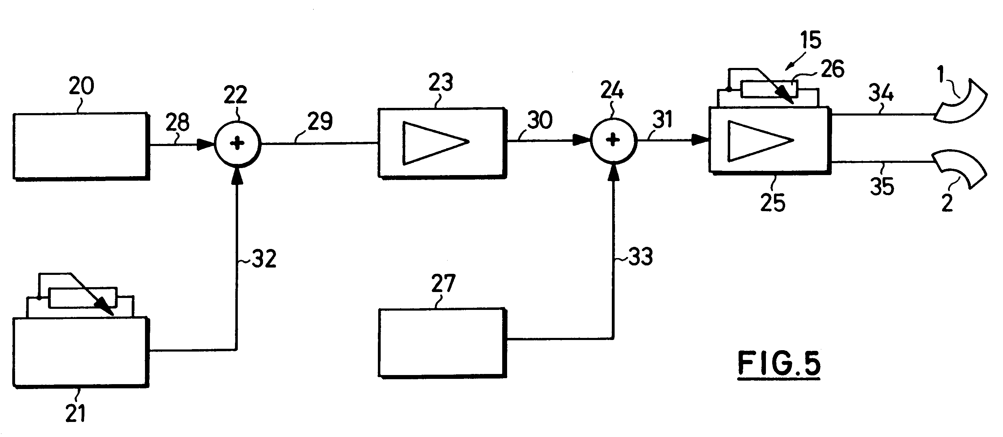

Eine Schaltungsanordnung zur Erzeugung eines niederfrequenten Wechselstroms, bei welcher ebenfalls drei Wechselströme von ca. 4 kHz überlagert werden, ist auch aus der EP-A-0 035 138 bekannt. Durch die Überlagerung der Wechselströme wird eine Schwebungsfrequenz erzeugt, die zwischen 0 und 100 Hz liegt. Dabei können sich anscheinend ebenfalls große und kleine Sinushalbwellen abwechseln (Fig. 5). Ein Polaritätswechsel der Schwebungsfrequenz ist jedoch nicht vorgesehen.

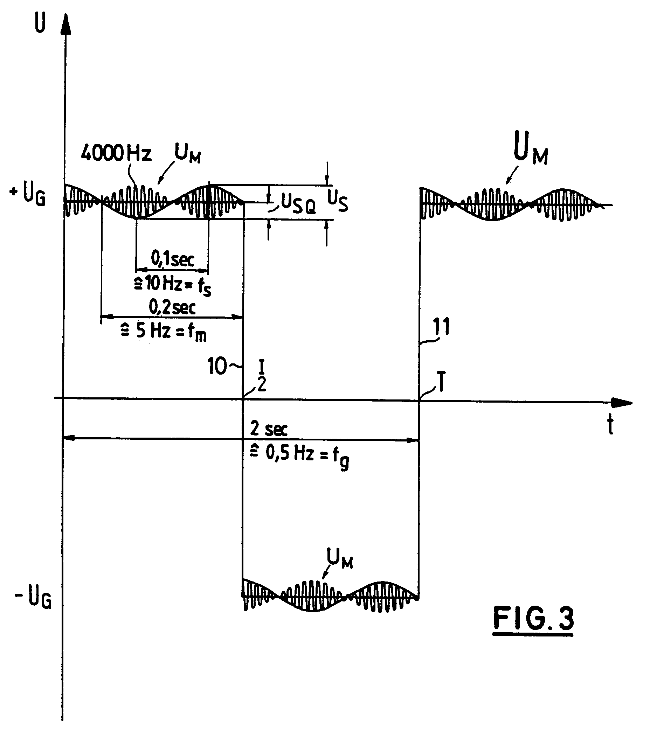

Es ist auch noch eine Vorrichtung zum Erzeugen elektrischer Ströme für die Einwirkung auf Muskeln bekannt, bei der insgesamt vier Wechselströme überlagert werden, und zwar zwei niederfrequente und zwei höherfrequente Ströme (CH-PS 624 850). Bei der ersten Überlagerung eines niederfrequenten mit einem höherfrequenten Strom (Fig. 3) ergibt sich eine gewöhnliche Zweiseitenbandmodulation (vgl. Meinke-Gundlach, a. a. O., O 2, Bild 1). Gleiches gilt für die Überlagerung eines zweiten niederfrequenten mit einem zweiten höherfrequenten Strom (Fig. 6), wobei lediglich die zweite höhere Frequenz niedriger als die erste höhere Frequenz und das zweite niederfrequente gegenüber dem ersten niederfrequenten Signal invertiert ist. Durch die Überlagerung der beiden Zweiseitenbandmodulationen (Fig. 7) ergibt sich eine Zweifachmodulation des Stroms mit der höchsten Frequenz, wobei die Amplitude dieses Stroms mit der nächsthöheren Frequenz und diese durch die niedrigste Frequenz moduliert ist. Nachteilig ist bei der doppelten Zweiseitenbandmodulation, daß der Stromverlauf symmetrisch zur Null-Linie verläuft, d. h. es findet kein Polaritätswechsel der Amplituden statt. Damit wird auch ein Muskel stets symmetrisch gereizt.

Schließlich ist auch eine Generatorschaltung bekannt, die durch geeignete Programmierung eines ROMs verschiedene Wechselspannungen erzeugen kann (A. H. Seidman, "Integrated Circuits Applications Handbook", John Wiley & Sons, 1983, Seiten 387 bis 389, Kapitel "12.8 Arbitrary Waveform Generator"). Es ist jedoch nicht offenbart, diese Generatorschaltung eine anspruchsgemäße Wechselspannung erzeugen zu lassen und diese Spannung an die Elektroden eines Elektrotherapiegeräts zu legen.

Der Erfindung liegt deshalb die Aufgabe zugrunde, einen Muskel über eine vorgegebene Zeit auf unterschiedliche Weise elektrisch zu reizen, damit eine einseitige Reizung entfällt.

Diese Aufgabe wird gemäß den Merkmalen des Patentanspruchs 1 gelöst.

Der mit der Erfindung erzielte Vorteil besteht insbesondere darin, daß durch den allmählichen Polaritätswechsel der Grundfrequenz der Muskel zunächst von der einen Seite und dann von der anderen Seite aus gleichstrommäßig gereizt wird, obgleich die pulsierende Schwebungsreizung beibehalten wird. Ist die Grundfrequenz eine niederfrequente Sinus was eine natürliche Regulation von blockierten Wirbelgelenken ermöglicht.

Ausführungsbeispiele der Erfindung sind in der Zeichnung dargestellt und werden im folgenden näher beschrieben. Es zeigen:

Fig. 1     eine Anordnung mit zwei Elektroden, einer Gleichspannungsquelle, einer Modulationsspannungsquelle und mehreren Umschaltern;

Fig. 2     eine graphische Darstellung einer Gleichspannung, der eine Schwebungsspannung überlagert ist;

Fig. 3     eine Darstellung gemäß Fig. 2, bei der fortwährend von positiver auf negative Polarität und umgekehrt geschaltet wird;

Fig. 4     eine niederfrequente Wechselstromkurve, der eine Schwebung überlagert ist;

Fig. 5     ein Blockschaltbild einer Anordnung für die Realisierung der Stromkurve gemäß Fig. 4.

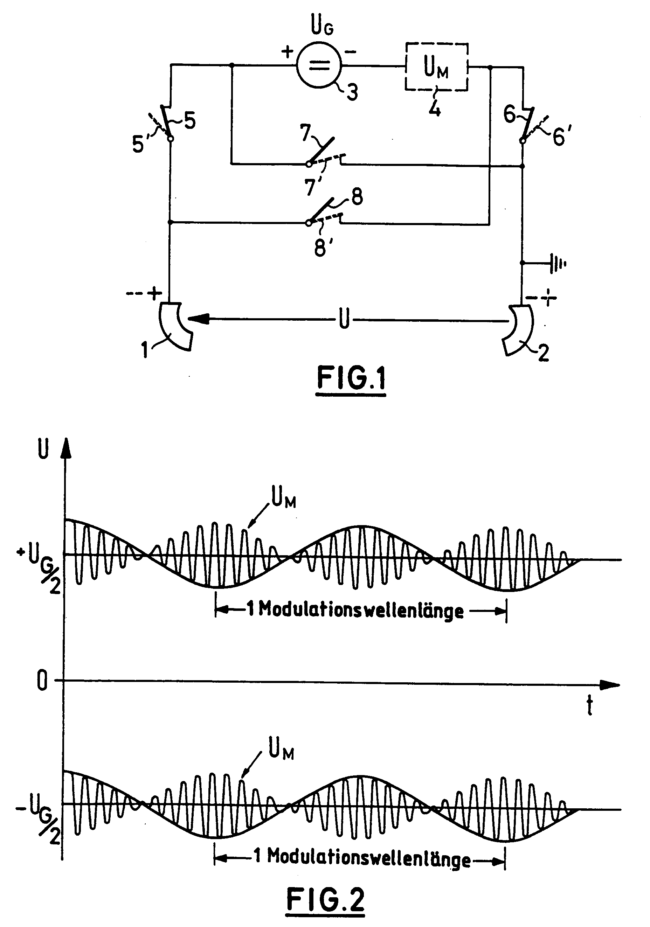

In der Fig. 1 sind zwei Handelektroden 1, 2 dargestellt, die z. B. von zwei Seiten an einen Muskel herangeführt werden, damit dieser Muskel zwischen den beiden Elektroden 1, 2 zu liegen kommt. Die Handelektroden werden mit einer Spannung U versorgt, die sich aus einer Gleichspannung $U_G$ und einer Modulationsspannung $U_M$ zusammensetzt. Bei der Modulationsspannung handelt es sich vorzugsweise um eine Schwebungsspannung, die durch Überlagerung zweier Wechselspannungen mit kleiner Frequenzdifferenz entsteht.

Die Gleichspannung $U_G$ wird von einer Gleichspannungsquelle 3 geliefert, zu der eine Modulationsspannungsquelle 4 in Reihe geschaltet ist. Die Summenspannung U gelangt über geschlossene Schalter 5, 6 auf die Elektroden 1, 2. Hierbei steht an der Elektrode 1 ein positives Potential an, weil diese Elektrode 1 mit dem Pluspol der Spannungsquelle 3 verbunden ist. An der Elektrode 2 liegt dagegen negatives Potential an weil diese Elektrode 2 über die Modulationsspannungsquelle 4 mit dem negativen Pol der Spannungsquelle 3 in Verbindung steht.

Mit Hilfe der Schalter 5, 6 und 7, 8 kann die Polarität den Elektroden 1, 2 vertauscht werden. Nehmen die Schalter 5 bis 8 diejenigen Postionen ein, die mittels der ausgezogenen Linien dargestellt sind, so liegen an der Elektrode 1 Plus- und an der Elektrode 2 Minuspotential. Nehmen die Schalter 5 bis 8 die mit gestrichelten Linien angedeuteten Positionen ein, so liegen an der Elektrode 2 Plus- und an der Elektrode 1 Minuspotential.

In der Fig. 2 sind die beiden an den Elektroden 1, 2 anstehenden Potentiale dargestellt, wobei die obere Hälfte der Fig. 2 das Potential an der Elektrode 1 und die untere Hälfte das Potential an der Elektrode 2 zeigt. Die beiden Potentiale sind symmetrisch zur Nullinie angeordnet. Wird eine der beiden Elektroden auf Nullpotential gelegt, was z. B. durch die Erdung der Elektrode 2 geschehen kann, ändert sich an den relativen Potentialen der beiden Elektroden 1, 2 nichts. Nach wie vor ist die Elektrode 1 positiver als die Elektrode 2. Statt $U_G/2$ liegt dann $U_G$ an der nicht geerdeten Elektrode; d. h. das Potential besteht dann aus U - OV = U , und es fließt ein entsprechender Gleichstrom mit überlagertem Wechselstrom von der Elektrode 1 zur Elektrode 2, wenn man die positive Zählrichtung wählt. Durch den Gleichstromanteil, der fortwährend in dieselbe Richtung fließt, wird der Muskel einseitig belastet. Diese einseitige Belastung kann auch nicht durch den symmetrisch am Muskel angreifenden Schwebestrom verringert werden. Durch Öffnen der Schalter 5, 6 und durch Schließen weiterer Schalter 7, 8 kann die Spannung umgepolt werden, so daß der Gleichstromanteil nun in die umgekehrte Richtung fließt. Die neuen Positionen der Schalter 5 bis 8 sind durch gestrichelte Schalter 5' bis 8' dargestellt.

Das im unteren Teil der Fig. 2 dargestellte Potential liegt nun an der Elektrode 1, während das im oberen Bereich dargestellte Potential an der Elektrode 2 liegt.

Es versteht sich, daß die in der Fig. 1 dargestellten Schalter durch Thyristoren, GTO-Thyristoren oder steuerbare Transistoren realisiert werden können.

In der Fig. 3 ist dargestellt, wie sich die Spannung U verändert, wenn die Schalter 5 bis 8 in bestimmten Zeitabständen umgeschaltet werden. Während einer Zeit T/2 liegt das positive Potential $U_G$ mit dem aufmodulierten Potential $U_M$ an der einen Elektrode 1, wohingegen die andere Elektrode 2 z. B. auf Erdpotential liegt, während in der darauffolgenden Zeit bis zum Zeitpunkt T das erwähnte positive Potential an der Elektrode 2 liegt und die Elektrode 1 auf Erdpotential gelegt ist. Dieser Polaritätswechsel wiederholt sich z. B. nach jeder Sekunde, so daß sich eine Wechselfrequenz von 0,5 Hz ergibt, die Grundfrequenz $f_g$ genannt sei. Der Wechselspannung mit der Grundfrequenz $f_g$ ist eine Schwebungsfrequenz überlagert, die z. B. 10 Hz beträgt und $f_s$ genannt sei. Die hierzu gehörende Modulationsfrequenz beträgt 5 Hz und wird $f_m$ genannt. Die Schwebungsbäuche haben eine Dicke von $U_s$ und entsprechen dem doppelten Betrag der Schwebungsamplitude $U_{sa}$. Die Abfall- und Anstiegsflanken 10, 11 sind ebenfalls mit einem Schwebungssignal moduliert, was jedoch in der Fig. 3 nicht dargestellt ist. Hierbei treten wegen der abrupten Stromänderungen auch noch Überlagerungen von Oberwellen auf, die eine exakte Darstellung ohnehin praktisch unmöglich machen. Die Fig. 2 ist somit eine quasi-idealisierte Darstellung, bei der angenommen wird, absolut steile Flanken 10, 11 seien möglich, was jedoch in Wirklichkeit nicht der Fall ist. Die Fig. 2 zeigt auch nicht die wahren Frequenzverhältnisse, sondern nur ihre prinzipielle Zuordnung.

Die Schwebungsfrequenz wird im vorliegenden Fall durch die Überlagerung einer Wechselspannung von 4000 Hz mit einer Wechselspannung von 3990 Hz erzeugt. Für $f_s$ gilt dann

$$f_s = f_1 - f_2 = 4000\ Hz - 3990\ Hz = 10\ Hz$$

Die Amplitude der Grundschwingung, d. h. $U_c$, ist bei einem bevorzugten Ausführungsbeispiel der Erfindung etwa doppelt so hoch wie die Amplitude $U_s$ der Schwebebäuche. Hierdurch wird gewährleistet, daß die Schwebebäuche durch die Sinuskurve in den positiven Bereich gehoben und von dort anschließend wieder in den negativen Bereich gesenkt werden.

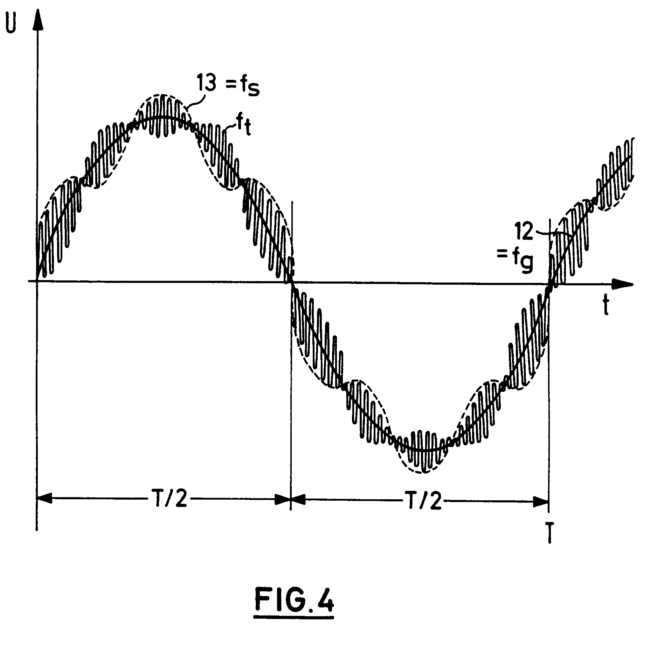

Die Grundfrequenz $f_g$ ist in der Fig. 3 als Rechteckschwingung dargestellt. Diese Rechteckschwingung kann mit Hilfe einer Fourier-Analyse in verschiedene Sinus- bzw. Cosinusschwingungen umgeformt werden. Diese Schwingungen enthalten auch eine Sinusschwingung mit der Frequenz $f_g$ (vgL z. B. E. Phllippow: Taschenbuch Elektrotechnik, Band 1, Allgemeine Grundlagen, 3. Auflage, 1986, S. 269). Statt einer Rechteckschwingung kann auch eine Dreieckschwingung oder eine andere Schwingung verwendet werden. Wichtig ist lediglich, daß diese Schwingung eine niederfrequente Grundschwingung besitzt. Verwendet man statt einer Rechteck- oder Dreieckschwingung deren Grundfrequenz als Basis, so ergibt sich die Spannungskurve nach Fig. 4.

In dieser Fig. 4 ist der Grundwelle 12 mit der Frequenz $f_g = 1/T$ die Schwebung 13 aufmoduliert, die ihrerseits durch die Schwebungsfrequenz $f_s$ und die Trägerfrequenz $f_t$ charakterisiert ist. Die Trägerfrequenz $f_t$ beträgt, wie bereits erwähnt, etwa 4000 Hz. Mit einer Spannung U gemäß Fig. 4 an den Elektroden 1, 2, d. h.

mit einem niederfrequenten Sinus, dem eine Schwebung überlagert ist, lassen sich überraschende therapeutische Wirkungen erzielen. Einerseits wird der behandelte Muskel mit einer Frequenz von etwa 4000 Hz beaufschlagt, welche auf die Schmerzrezeptoren der Haut die geringsten Auswirkungen hat. Eine Frequenz zwischen etwa 3000 Hz und 4000 Hz wird deshalb von den Patienten als angenehm empfunden. Anderseits erfolgt die Zuführung der Frequenz zwischen 3000 Hz und 4000 Hz mit Hilfe der Schwingungsbäuche einer Schwebung. Auch diese Schwebung, die in einer kontinuierlichen Zunahme und anschließenden Abnahme der Amplitude der Trägerfrequenz $f_t$ besteht, hat einen positiven Einfluß auf das Gewebe, was aus der sogenannten Interferenzstrom-Therapie bereits bekannt ist.

Entscheidend ist indessen, daß die Spannung der Schwebungskurve einmal an der einen Seite des Muskels und einmal an der anderen Seite des Muskels angreift. Dieser Effekt wird durch den relativ langsamen Polaritätswechsel der Grundfrequenz $f_g$ erreicht. Durch den langsamen Polaritätswechsel erreicht man praktisch dieselben Effekte wie bei einer Gleichstromtherapie, ohne jedoch deren Nachteile in Kauf nehmen zu müssen. Außerdem bleiben die Vorteile der Wechselstromtherapie voll erhalten.

In der Fig. 5 ist ein Blockschaltbild einer Anordnung für die Erzeugung einer Spannungskurve gemäß Fig.4 dargestellt.

Man erkennt hierbei zwei Sinusgeneratoren 20 und 21, von denen der eine Generator 20 eine Spannung von 4000 Hz erzeugt, während der andere Generator eine Spannung mit einer Frequenz von 3990 zur Verfügung stellt. Über die Leitungen 28 bzw. 32 werden die beiden Spannungen auf eine Mischstufe 22 gegeben, wo die Überlagerung stattfindet, so daß sich eine Schwebungsspannung ergibt. Unter Mischstufe wird hierbei eine Einrichtung für die allgemeine z. B. additive oder multiplikative Verknüpfung bzw. Überlagerung von Kurven verstanden und nicht nur ein Frequenzumsetzer, wie er etwa bei einem Überlagerungsempfänger zwischen HF-Vorstufe und Zwischenfrequenzverstärker vorgesehen ist. Diese Schwebungsspannung wird über eine Leitung 29 auf einen Schwebungsverstärker 23 gegeben, der die Schwebungsspannung verstärkt und auf einen Modulator 24 gibt. Das verstärkte Schwebungssignal, welches über eine Leitung 30 auf den Modulator 24 gelangt, wird mit einer Sinusspannung von etwa 0,1 bis 1 Hz moduliert, die aus einem Sinussignalgeber 27 kommt. Von hier aus gelangt das modulierte Signal über eine Leitung 31 auf einen Endverstärker 25, der über einen Widerstand 26 regelbar ist. Am Ausgang des Endverstärkers 25 mit den Leitungen 34, 35 sind die beiden Elektroden 1, 2 verbunden. Die Amplitude des Oszillators 21 ist über einen Widerstand 36 regelbar. Hierdurch kann erreicht werden, daß die Amplituden der Spannungen der beiden Oszillatoren stets gleich sind, was für den Sehwebungseffekt von besonderer Bedeutung ist. Der Verstärker 23 wird deshalb zwischen den beiden Mischstufen 22, 24 angeordnet, um das Verhalten zwischen der Amplitude der Grundschwingung und der Amplitude des Schwebungsbands einstellen zu können Wie bereits erwähnt, ist ein Amplitudenverhalten von 2 : 1 besonders vorteilhaft.

Anstelle einer einfachen Schwebung kann der Sinusgrundkurve 12 auch eine Schwebung überlagert werden, die einer Zweiseitenband-Amplitudenmodulation mit unterdrücktem Träger entspricht.

Die in der Fig. 5 dargestellte Anordnung ist nur eine von mehreren Realisierungsmöglichkeiten. Die Überlagerung der beiden exakt harmonischen Spannungen der Oszillatoren 20, 21 entspricht einer amplitudenmodulierten Welle mit einer einzigen Modulationsfrequenz. Es ist deshalb grundsätzlich möglich, die zahlreichen auf dem Gebiet der Amplitudenmodulation bekannten Modulatoren für die Erzeugung der Schwebungen zu verwenden, denn die amplitudenmodulierten Schwingungen sind der Überlagerung zweier exakt harmonischer Partialschwingungen äquivalent.

Es versteht sich außerdem, daß die in den Figuren 2 bis 4 dargestellten analogen Kurven unter Anwendung des Abtasttheorems digitalisiert werden können. Um eine Kurve gemäß Fig. 4 herzustellen, kann man diese Kurve mit wenigstens der doppelten Frequenz der höchsten in dieser Kurve vorkommenden Frequenz abtasten und die hieraus resultierenden Amplitudenproben in einzelne bits auflösen Diese bits können in einen ROM-Speicher abgelegt werden, der dann gewissermaßen die Kurvenform der Fig. 4 abspeichert. Mittels geeigneter Taktgeber kann hierauf diese Kurvenform wiederholt aus dem Speicher abgerufen werden.

Es versteht sich weiterhin, daß anstelle der externen Erzeugung der Kurvenform gemäß Fig. 4 auch eine interne Erzeugung möglich ist. In diesem Fall werden von außen verschiedene Wellen dem Muskel zugeführt, die dann erst am Muskel selbst die Kurvenform gemäß Fig. 4 ergeben.

Besonders hervorzuheben ist, daß der therapeutische Effekt besonders deutlich hervortritt, wenn das Verhältnis der Amplitude der Hüllkurve der Schwebung und der Amplitude des Trägersinus minimal 1 : 1 ist und maximal 1 : 2 ist. Wichtig ist auch, daß die Schwebungskurve durch Überlagerung zweier Spannungen erzeugt wird, die im Spektrum zwischen 1 und 5.000 Hz liegen.

**Patentansprüche**

1. Elektrotherapie-Gerät mit Elektroden, zwischen denen ein zu behandelndes Gewebe angeordnet werden kann, wobei zwischen den Elektroden eine Wechselspannung (13) mit einer Frequenz $f_s$ zwischen 1 Hz und 100 Hz liegt, **dadurch gekennzeichnet**, daß diese Wechselspannung (13) die Einhüllende eines Wechselfeldes ($U_M$) mit einer Frequenz von ca. 4000 Hz ist und daß eine Wechselspannung (12) mit einer Frequenz $f_g$ zwischen 0,1 und 5 Hz linear überlagert ist, wobei $f_g < f_s$ gilt und die Amplitude der Wechselspannung (13) mit der Frequenz $f_s$ nicht größer als die Amplitude der Wechselspannung (12) mit der Frequenz $f_g$ ist.

2. Elektrotherapie-Gerät nach Anspruch 1, **dadurch gekennzeichnet**, daß die Wechselspannung (12) mit einer Frequenz $f_g$ zwischen 0,1 und 5 Hz eine Sinus- oder Cosinuswechselspannung ist.

3. Elektrotherapie-Gerät nach Anspruch 1, **dadurch gekennzeichnet**, daß die Wechselspannung (13) eine Schwebungsspannung ist, die durch die Überlagerung zweier Spannungen mit eng benachbarten Frequenzen bei ca. 4000 Hz, z. B. 4000 Hz und 3990 Hz, gebildet ist.

4. Elektrotherapie-Gerät nach Anspruch 3, **dadurch gekennzeichnet**, daß die Überlagerung in einem ersten Mischer (22) vorgenommen wird, dem die Ausgangsspannungen eines ersten und eines zweiten Oszillators (20, 21) zugeführt sind.

5. Elektrotherapie-Gerät nach Anspruch 4, **dadurch gekennzeichnet**, daß der Ausgang des ersten Mischers (22) auf einen zweiten Mischer (24) gegeben ist, dem auch das Ausgangssignal eines Wechselspannungsgenerators (27) zugeführt ist, das eine Frequenz zwischen 0,1 und 5 Hz hat.

6. Elektrotherapie-Gerät nach Anspruch 5, **dadurch gekennzeichnet**, daß zwischen dem ersten Mischer (22) und dem zweiten Mischer (24) ein Verstärker (23) vorgesehen ist.

7. Elektrotherapie-Gerät nach Anspruch 5, **dadurch gekennzeichnet**, daß der Ausgang des zweiten Mischers (24) mit einem regelbaren Verstärker (25) verbunden ist, dessen Ausgang mit den Elektroden (1, 2) in Verbindung steht.

8. Elektrotherapie-Gerät nach Anspruch 1 und Anspruch 3, **dadurch gekennzeichnet**, daß die Amplitude ($U_G$ bzw. $U_{G/2}$) der Wechselspannung (12) mit der Frequenz zwischen 0,1 und 5 Hz etwa doppelt so groß ist wie der Bauch ($U_S$) einer Schwebung.

9. Elektrotherapie-Gerät nach Anspruch 3, **dadurch gekennzeichnet**, daß die Amplituden der beiden überlagerten Spannungen gleich groß sind.

## Claims

1. Electrotherapy device with electrodes, between which a tissue to be treated can be arranged, an alternating voltage (13) with a frequency $f_s$ of between 1 Hz and 100 Hz being present between the electrodes, characterised in that this alternating voltage (13) is the envelope of an alternating field ($U_M$) with a frequency of approximately 4000 Hz, and in that an alternating voltage (12) with a frequency $f_g$ of between 0.1 and 5 Hz is linearly superimposed, where $f_g < f_s$ and the amplitude of the alternating voltage (13) with the frequency $f_s$ is no greater than the amplitude of the alternating voltage (12) with the frequency $f_g$.

2. Electrotherapy device according to Claim 1, characterised in that the alternating voltage (12) with a frequency $f_g$ of between 0.1 and 5 Hz is a sinusoidal or cosinusoidal alternating voltage.

3. Electrotherapy device according to claim 1, characterised in that the alternating voltage (13) is a beat voltage which is formed by the superimposition of two voltages with frequencies which are close together and are at approximately 4000 Hz, for example 4000 Hz and 3990 Hz.

4. Electrotherapy device according to Claim 3, characterised in that the superimposition is carried out in a mixer (22) which is supplied with the output voltages of a first and a second oscillator (20, 21).

5. Electrotherapy device according to Claim 4, characterised in that the output of the first mixer (22) is transmitted to a second mixer (24) which is also supplied with the output signal of an alternating-voltage generator (27) having a frequency of between 0.1 and 5 Hz.

6. Electrotherapy device according to Claim 5, characterised in that an amplifier (23) is provided between the first mixer (22) and the second mixer (24).

7. Electrotherapy device according to Claim 5, characterised in that the output of the second mixer (24) is connected to a regulable amplifier (25), the output of which is connected to the electrodes (1, 2).

8. Electrotherapy device according to Claim 1 and Claim 3, characterised in that the amplitude ($U_G$ or $U_{G/2}$) of the alternating voltage (12) with the frequency of between 0.1 and 5 Hz is approximately twice as large as the antinode ($U_S$) of a beat.

9. Electrotherapy device according to Claim 3, characterised in that the amplitudes of the two superimposed voltages are of the same size.

## Revendications

1. Appareil d'électrothérapie muni d'électrodes entre lesquelles peut être disposé un tissu à traiter, une tension alternative (13) étant appliquée entre les électrodes à une fréquence $f_s$ de l'ordre de 1 Hz à 100 Hz, caractérisé en ce que cette tension alternative (13) constitue l'enveloppe d'un champ alternatif ($U_M$) d'une fréquence d'environ 4000 Hz et en ce qu'une tension alternative (12) est superposée linéairement à une fréquence $f_g$ entre 0,1 et 5 Hz, $f_g < f_s$ étant appliquée et l'amplitude de la tension alternative (13) d'une fréquence $f_s$ n'étant pas supérieure à l'amplitude de la tension alternative (12) de la fréquence $f_g$.

2. Appareil d'électrothérapie selon la revendication 1, caractérisé en ce que la tension alternative (12) présentant une fréquence $f_g$ entre 0,1 et 5 Hz, constitue une tension alternative Cosinus ou Sinus.

3. Appareil d'électrothérapie selon la revendication 1, caractérisé en ce que la tension alternative (13) est une tension de battement qui est formée par la superposition de deux tensions présentant des fréquences très voisines d'environ 4000 Hz, par exemple 4000 Hz et 3990 Hz.

4. Appareil d'électrothérapie selon la revendication 3, caractérisé en ce que la superposition est effectuée dans un premier mélangeur (22) auquel sont appliquées les tensions de sortie d'un premier et d'un second oscillateurs (20, 21).

5. Appareil d'électrothérapie selon la revendication 4, caractérisé en ce que la sortie du premier mélangeur (22) est fournie à un second mélangeur (24) qui reçoit également, de la part d'un générateur de tension alternative (27), le signal de sortie dont la fréquence est de l'ordre de 0,1 et 5 Hz.

6. Appareil d'électrothérapie selon la revendication 5, caractérisé en ce qu'un amplificateur (23) est prévu entre le premier mélangeur (22) et le second mélangeur (24).

7. Appareil d'électrothérapie selon la revendication 5, caractérisé en ce que la sortie du second mélangeur (24) est reliée à un amplificateur réglable (25) dont la sortie est connectée aux électrodes (1, 2).

8. Appareil d'électrothérapie selon la revendication 1 et la revendication 3, caractérisé en ce que l'amplitude ($U_G$ respectivement $U_{G/2}$) de la tension alternative (12), qui présente la fréquence entre 0,1 et 5 Hz, est deux fois plus grande que le ventre ($U_S$) d'un battement.

9. Appareil d'électrothérapie selon la revendication 3, caractérisé en ce que les amplitudes des deux tensions superposées sont identiques.

EP 0 612 259 B1

FIG.1

FIG.2

8

FIG.3

FIG.4

FIG.5